Europäisches Patentamt

European Patent Office

Office européen des brevets

(19)

(11) **EP 1 125 123 B1**

(12) **FASCICULE DE BREVET EUROPEEN**

(45) Date de publication et mention
de la délivrance du brevet:
**04.08.2004   Bulletin 2004/32**

(21) Numéro de dépôt: **99949096.4**

(22) Date de dépôt: **26.10.1999**

(51) Int Cl.⁷: **G01N 31/22**

(86) Numéro de dépôt international:
**PCT/FR1999/002592**

(87) Numéro de publication internationale:
**WO 2000/025130 (04.05.2000 Gazette 2000/18)**

(54) **DISPOSITIF DE CONTROLE DES CARACTERISTIQUES D'UN LIQUIDE DE REFROIDISSEMENT ET PROCEDE DE MISE EN OEUVRE**

VORRICHTUNG UND VERFAHREN ZUR PRÜFUNG DER EIGENSCHAFTEN EINER KÜHLFLÜSSIGKEIT

DEVICE FOR CONTROLLING THE CHARACTERISTICS OF A COOLING LIQUID AND METHOD USING SAME

(84) Etats contractants désignés:
**AT BE CH CY DE DK ES FI FR GB GR IE IT LI LU MC NL PT SE**

(30) Priorité:  **28.10.1998  FR 9813515**

(43) Date de publication de la demande:
**22.08.2001   Bulletin 2001/34**

(73) Titulaire: **Total France**
**92800 Puteaux (FR)**

(72) Inventeurs:
• **GENET, Nicole**
**F-78110 Le Vésinet (FR)**
• **HAIUN, Roger**
**F-94380 Bonneuil sur Marne (FR)**

(74) Mandataire: **Cabinet Hirsch**
**58, avenue Marceau**
**75008 Paris (FR)**

(56) Documents cités:
**US-A- 3 973 909          US-A- 5 091 081**
**US-A- 5 496 739          US-A- 5 529 751**
**US-A- 5 744 365**

• **PATENT ABSTRACTS OF JAPAN vol. 008, no. 163 (P-290), 27 juillet 1984 (1984-07-27) & JP 59 060356 A (NISSAN DIESEL KOGYO KK;OTHERS: 01), 6 avril 1984 (1984-04-06)**
• **PODDA F ET AL: "MESURE COLORIMETRIQUE DE L'ALCALINITE" COMPTES RENDUS DE L ACADEMIE DES SCIENCES: SERIE II: SCIENCES DE LA TERRE ET DES PLANETES, vol. 319, no. 6, 22 septembre 1994 (1994-09-22), pages 651-657, XP000477248 paris, fr**

## Description

La présente invention concerne un dispositif simple et transportable de contrôle de la qualité d'un liquide de refroidissement, notamment par rapport à sa température de protection et à sa réserve d'alcalinité. Elle concerne également le procédé de mise en oeuvre du dit dispositif.

On entend par dispositif simple et transportable, un appareil de faibles dimensions qu'un opérateur peut utiliser en suivant des consignes opératoires pour contrôler la qualité d'un fluide de refroidissement neuf ou usagé, sans avoir de connaissances techniques particulières, notamment chimiques.

Jusqu'à présent pour suivre l'évolution ou la qualité d'un fluide de refroidissement on utilisait soit un pèse-glycol soit un réfractomètre pour déterminer la température de protection. Pour doser les inhibiteurs de corrosion, les techniques utilisées étaient essentiellement des méthodes de laboratoires telles que la chromatographie liquie ou l'électrophorèse, difficilement transportables sur site d'exploitation, et pas du tout adaptées aux hommes de terrains, par exemple les garagistes ou les personnes en charge du bon fonctionnement des moteurs industriels ou des moteurs d'un parc de voitures, de camions ou de bus.

En outre, ces mesures, fort appropriées pour un liquide de refroidissement de type minéral ne conviennent plus à des liquides de refroidissement dits de technologie toute organique, obtenus à partir d'antigels concentrés contenant des mélanges à base d'acides carboxyliques aux propriétés anti-corrosives pour lesquels la mesure de la seule température de protection est insuffisante pour en contrôler la qualité.

Pour ce nouveau type de liquide de refroidissement tout organique, il est usuel de caractériser le niveau de performance du liquide de refroidissement par sa réserve d'alcalinité, permettant de prévoir son pouvoir anticorrosion.

Pour mesurer cette réserve d'alcalinité, ces tests acidobasiques sont réalisés par potentiométrie conduisant à l'obtention d'une courbe de neutralisation d'un volume connu de liquide de refroidissement par un acide, souvent par l'acide chlorhydrique faiblement concentré (par exemple à 0,1 N)et on note le volume d'acide nécessaire au point d'inflexion de la courbe. Ce volume ramené à 10 ml d'antigel correspond à la réserve d'alcalinité du liquide de refroidissement.

Cette technique bien que très précise n'est pas transposable pour une mesure par un opérateur n'ayant pas de connaissances techniques particulières.

La présente invention vise donc un dispositif simple et transportable pour un contrôle fiable des caractéristiques d'un liquide de refroidissement, pouvant être utilisé par un opérateur n'ayant pas de connaissances techniques particulières mais ayant les capacités pour suivre une suite de consignes opératoires simples.

La présente invention a donc pour objet un dispositif de contrôle de la température de protection et de la réserve d'alcalinité d'un liquide de refroidissement, obtenu par dilution d'un antigel contenant des composés carboxyliques, comprenant au moins un réfractomètre, du papier pH et une éprouvette, l'ensemble étant enfermé dans une enceinte transportable, caractérisé en ce que l'éprouvette est graduée en température de protection, chaque graduation correspondant à un volume X de liquide de refroidissement présentant une température de protection connue variant avec le taux de dilution C du dit antigel dans l'eau, et une réserve alcaline minimale désirée, X correspondant à la formule (I) ci-après :

$$X = \frac{10 \times V_A}{RA \times C} \tag{I}$$

avec $V_A$ correspondant au volume d'acide nécessaire à la neutralisation de la réserve d'alcalinité RA et C la concentration en antigel du liquide de refroidissement, exprimée en pourcentage.

La présente invention a comme deuxième objet le procédé de mise en oeuvre du dispositif transportable pour effectuer la mesure des caractéristiques du liquide de refroidissement que ce liquide soit neuf ou usagé, notamment sa température de protection et sa réserve d'alcalinité.

Ce procédé de mise en oeuvre selon l'invention consiste à mesurer de façon consécutive,

a) la température de protection du dit liquide,
b) le pH du dit liquide,
c) et la réserve d'alcalinité RA du dit liquide par un test colorimétrique acido-basique visuel,

le dit procédé étant caractérisé en ce que le dit test colorimétrique consiste à prélever dans l'éprouvette graduée un volume X de liquide de refroidissement correspondant à la température de protection mesurée, à ajouter dans la dite éprouvette de 5 à 15 ml d'eau, puis de 2 à 8 ml d'un acide fort de 0.05 à 0.2N au moyen d'une pipette, à ajouter quelques gouttes de colorant et à observer le virage de couleur.

L'avantage de ce dispositif et de son procédé de mise en oeuvre est de permettre à un opérateur peu qualifié et qui a peu de connaissances techniques d'utiliser cet appareil sans avoir à calculer les volumes de liquide de refroidissement, d'acide et d'eau nécessaires au test colorimétrique, les mesures de pH et de température de protection ne demandant que quelques manipulations simples.

Un autre avantage est de disposer d'un outil transportable et fiable car les tests à mettre en oeuvre sont suffisamment sensibles pour caractériser la qualité d'un fluide de refroidissement.

Dans un mode de mise en oeuvre particulier du procédé, l'acide utilisé pour le test colorimétrique est un acide minéral du groupe constitué par l'acide chlorhydrique, l'acide sulfurique, l'acide nitrique, l'acide chlorhydrique étant préféré.

Le colorant utilisé pour le test colorimètrique est choisi de préférence pour une gamme de virage du pH compris entre 3 et 4,6. Il est choisi parmi les colorants de type bleu de bromophénol , bleu de bromochlorophénol, rouge congo ou orangé de méthyle. Par exemple, le bleu de bromophénol vire au jaune pour un pH inférieur à 3.5, et devient bleu violet pour un pH supérieur à 4. Pour faciliter la manipulation d'acide, on peut ajouter quelques gouttes de colorant à l'acide qui de transparent devient jaune.

En outre, dans le procédé selon l'invention, chaque étape dans la suite des opérations de (a) à (c) permet d'accepter ou de refuser le liquide de refroidissement, selon une procédure d'utilisation, le liquide de refroidissement aux caractéristiques acceptables étant celui qui a franchi successivement les étapes a, b et c dans cet ordre, avec succès.

Le dispositif selon l'invention est particulièrement adapté au contrôle de qualité des liquides de refroidissement obtenus à partir d'antigels contenant essentiellement des composés carboxyliques, sous forme acide, ester ou amide .

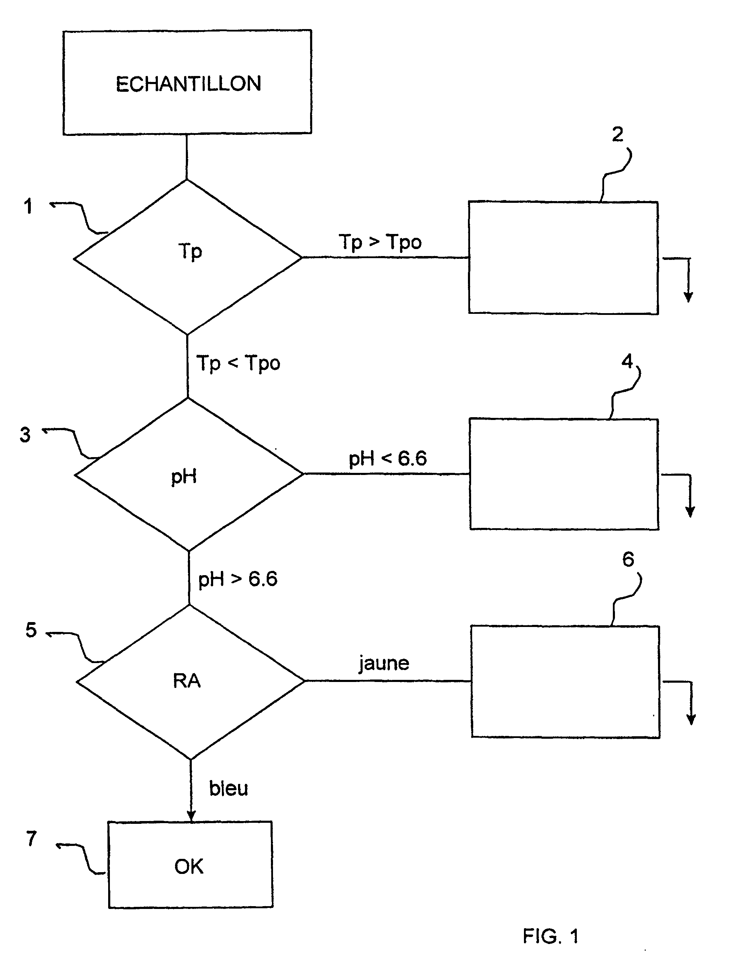

La figure 1 présente une illustration logique du procédé selon l'invention, constituant le mode opératoire à mettre en oeuvre.

Quelques gouttes de l'échantillon prélevé de liquide de refroidissement sont déposées sur le prisme du réfractomètre (1), ici un réfractomètre ATAGO vendu par la société BIOBLOCK, puis la température de protection (Tp) est lue. Si Tp est supérieure à une température de protection correspondant à la limite acceptable pour le liquide de refroidissement (Tpo), le liquide de refroidissement est rejeté(2). Si Tp est inférieur ou égal à Tpo, le pH du liquide est mesuré au moyen du papier pH (pH détecté variant de 6 à 8.1) trempé dans l'échantillon de liquide de refroidissement (3). Si le pH est inférieur à 6.6, le liquide de refroidissement est rejeté (4). Dans le cas contraire, c'est-à-dire que le pH est supérieur ou égal à 6.6, on peut pratiquer le test colorimètrique (5) caractéristique de la réserve d'alcalinité du liquide. Dans ce test, il s'agit de remplir l'éprouvette graduée en température de protection par le liquide de refroidissement jusqu'au trait correspondant à la température de protection mesurée, d'ajouter de 5 à 15 ml d'eau, le volume nécessaire d'acide selon la concentration utilisée entre 2 et 8 ml, à ajouter quelques gouttes de colorant et à observer visuellement le virage de couleur du mélange. Si, dans le cas du bleu de bromophénol, le mélange vire au jaune, le liquide de refroidissement est à rejeter (6), s'il reste bleu, le liquide de refroidissement présente toutes les qualités requises de température de protection et de réserve d'alcalinité. Bien entendu, toute une gamme de couleurs de virage est fournie avec le dispositif de l'invention pour apprécier les limites de virage de couleur acceptables.

Dans la suite de la présente description, des exemples sont donnés à titre d'illustrations de l'invention, ne visant pas à en limiter la portée.

EXEMPLE I

Le présent exemple vise à décrire la méthode suivie pour graduer l'éprouvette objet de la présente invention.

Pour viser une réserve d'alcalinité de 30 dans un liquide de refroidissement dont la concentration en antigel est C %, le volume X à prélever pour une neutralisation avec 6 ml d'acide chlorhydrique 0.1N est donc :

$$X = \frac{10 \times 6}{30 \times C} = 2/C$$

Comme à chaque concentration d'antigel dans l'eau correspond une température de protection Tp, il est possible d'établir le tableau I de correspondance suivant pour graduer l'éprouvette en température de protection.

TABLEAU I

| C (%) | Tp (°C) | X (ml) |
|---|---|---|
| 25 | -13 | 8 |
| 30 | -17 | 6.7 |
| 35 | -20 | 5.8 |

TABLEAU I   (suite)

| C (%) | Tp (°C) | X (ml) |
|---|---|---|
| 40 | -26 | 5 |
| 50 | -37 | 4 |

Ainsi pour une température de protection mesurée, l'opérateur peu qualifié n'a plus qu'à verser le liquide de refroidissement dans l'éprouvette graduée jusqu'au trait indiquant cette température de protection pour pratiquer le test colorimétrique.

EXEMPLE II

Le présent exemple vise à démontrer la fiabilité du test colorimétrique pour déterminer la réserve d'alcalinité d'un liquide de refroidissement par rapport à une méthode potentiométrique au cours d'un test simple acido-basique.
On va ainsi préparer différents liquides de refroidissement contenant différentes concentrations de deux antigels dont la réserve d'alcalinité est pour l'un supérieure à 30 et pour l'autre inférieure à 30.
Pour effectuer le test colorimétrique on utilise une éprouvette graduée comme décrit dans l'exemple I. On verse pour une température de protection mesurée le volume correspondant de liquide de refroidissement dans l'éprouvette graduée puis on ajoute 10 ml d'eau, 6 ml d'acide chlorhydrique 0.1N, puis quelques gouttes de bleu de bromophénol.
Parallèlement, on prélève un volume de 5 à 10 ml de chaque liquide de refroidissement dans un récipient, puis on introduit une électrode de référence raccordée à un potentiomètre enregistreur, par exemple TIM 90 commercialisé par Tacusel, puis on introduit progressivement de l'acide chlorhydrique 0.1N dans le dit récipient en enregistrant la courbe de potentiel en fonction du volume d'acide versé. Après neutralisation, on note sur la courbe le volume d'acide introduit au point d'inflexion pour déterminer la réserve d'alcalinité du liquide étudié.
Les résultats de ces tests sont donnés dans le tableau II ci-après.

TABLEAU II

| Tp (°C) | C (%) | Antigel A | | Antigel B | |
|---|---|---|---|---|---|
| | | colorimétrie | potentiométrie | colorimétrie | potentiomètrie |
| - 13 | 25 | jaune | 26 | bleu | 34 |
| - 18 | 30 | jaune | 27 | bleu | 33 |
| - 26 | 40 | jaune | 28 | bleu | 35 |
| - 37 | 50 | jaune | 26 | bleu | 35 |

Il est évident d'après ces résultats que la méthode par colorimétrie permet d'accéder aux mêmes résultats que la méthode potentiométrique, mais de façon plus simple.

**Revendications**

1.  Dispositif de contrôle de la température de protection et de la réserve d'alcalinité d'un liquide de refroidissement, obtenu par dilution d'un antigel contenant des composés carboxyliques, comprenant au moins un réfractomètre, du papier pH et une éprouvette dans une enceinte fermée transportable, **caractérisé en ce que** l'éprouvette est graduée en température de protection, chaque graduation correspondant à un volume X de liquide de refroidissement présentant une température de protection connue variant avec le taux de dilution C du dit antigel dans l'eau, et une réserve alcaline minimale désirée, X correspondant à la formule (I) ci-après :

$$X = \frac{10 \times V_A}{RA \times C} \qquad (I)$$

avec $V_A$ correspondant au volume d'acide nécessaire à la neutralisation de la réserve d'alcalinité RA et C la concentration en antigel du liquide de refroidissement, exprimée en pourcentage.

**2.** Procédé de mise en oeuvre du dispositif selon la revendication 1 qui consiste à mesurer de façon consécutive,

a) la température de protection du dit liquide,
b) le pH du dit liquide
c) et la réserve d'alcalinité RA du dit liquide par un test colorimétrique acido-basique visuel,

le dit procédé étant **caractérisé en ce que** le dit test colorimétrique consiste à prélever dans l'éprouvette graduée un volume X de liquide de refroidissement correspondant à la température de protection mesurée, à ajouter dans la dite éprouvette de 5 à 15 ml d'eau, puis à introduire de 2 à 8 ml d'un acide fort de 0.05 à 0.2N, à ajouter quelques gouttes de colorant et à observer le virage de couleur.

**3.** Procédé selon la revendication 2 **caractérisé en ce que** l'acide fort est choisi dans le groupe constitué par l'acide chlorhydrique, l'acide sulfurique, l'acide nitrique, l'acide chlorhydrique étant préféré.

**4.** Procédé selon la revendication 2 **caractérisé en ce que** le colorant présente une gamme de virages du pH entre 3 et 4,6.

**5.** Procédé selon l'une des revendications 2 à 4 **caractérisé en ce que** le colorant est choisi parmi le bleu de bromophénol, le bleu de bromochlorophénol, le rouge congo et l'orangé de méthyle.

**6.** Procédé selon la revendication 2 **caractérisé en ce que** chaque étape dans la suite des opérations de (a) à (c) permet d'accepter ou de refuser le liquide de refroidissement, le liquide de refroidissement aux caractéristiques acceptables étant celui qui a franchi successivement les étapes a, b et c dans cet ordre, avec succès.

**Patentansprüche**

**1.** Vorrichtung zur Kontrolle der Schutztemperatur und der Basizitätsreserve einer Kühlflüssigkeit, erhalten durch Verdünnen eines Frostschutzmittels, das Carbonsäurenzusammensetzungen enthält, wobei sie mindestens ein Refraktometer, pH-Papier und ein Reagenzglas in einem transportierbaren geschlossenen Behälter umfasst, **dadurch gekennzeichnet, dass** das Reagenzglas bezüglich der Schutztemperatur geeicht ist, wobei jede Unterteilung einem Volumen X der Kühlflüssigkeit, die eine bekannte Schutztemperatur, die sich mit der Verdünnungsrate C des Frostschutzmittels in Wasser ändert, darstellt, und einer erwünschten minimalen basischen Reserve entspricht, wobei X der folgenden Formel (I) entspricht:

$$X = \frac{10 \times V_A}{RA \times C} \qquad \text{(I)},$$

wobei $V_A$ einem zur Neutralisation der Basizitätsreserve RA benötigten Volumen und C der Konzentration des Frostschutzmittels der Kühlflüssigkeit, ausgedrückt in Prozent, entspricht.

**2.** Verfahren zur Verwendung der Vorrichtung nach Anspruch 1, das darin besteht, auf folgende Art und Weise

a) die Schutztemperatur der Flüssigkeit,
b) den pH der Flüssigkeit
c) und die Basizitätsreserve RA der Flüssigkeit mittels eines visuellen kalorimetrischen Säure-Base-Tests zu messen,

wobei das Verfahren **dadurch gekennzeichnet ist, dass** der kalorimetrische Test aus der Entnahme eines Volumen X der Kühlflüssigkeit, das der gemessenen Schutztemperatur entspricht, aus dem geeichten Reagenzglas, Hinzugeben von 5 bis 15 ml Wasser in das Reagenzglas, anschließendem Einspeisen von 2 bis 8 ml einer 0.05 bis 0.2 N starken Säure, Hinzugeben einiger Tropfen eines Farbstoffes und Beobachten des Farbwechsels, besteht.

**3.** Verfahren nach Anspruch 2, **dadurch gekennzeichnet, dass** die starke Säure aus der Gruppe, die aus Salzsäure, Schwefelsäure, Salpetersäure besteht, ausgewählt wird, wobei Salzsäure bevorzugt ist.

**4.** Verfahren nach Anspruch 2, **dadurch gekennzeichnet, dass** der Farbstoff einen pH-Kurvenbereich zwischen 3 und 4.6 aufweist.

**5.** Verfahren nach einem der Ansprüche 2 bis 4, **dadurch gekennzeichnet, dass** der Farbstoff unter Bromphenolblau, Bromchlorphenolblau, Kongorot und Methylorange ausgewählt wird.

**6.** Verfahren nach Anspruch 2, **dadurch gekennzeichnet, dass** es jeder Schritt in der Abfolge der Operationen (a) bis (c) erlaubt, die Kühlflüssigkeit anzunehmen oder abzulehnen, wobei die Kühlflüssigkeit mit akzeptablen Merkmalen diejenige ist, die aufeinanderfolgend die Schritte a, b und c in dieser Reihenfolge mit Erfolg bestanden hat.

**Claims**

**1.** A device for checking the protection temperature and reserve alcalinity of a cooling liquid, obtained by diluting an anti-freeze containing carboxylic compounds, comprising at least one refractometer, pH paper and a test tube in a transportable closed enclosure, **characterised in that** the test tube is graduated in protection temperature, each graduation corresponding to a volume X of cooling liquid having a known protection temperature varying with the rate of dilution C of said anti-freeze in water, and a desired minimum alkaline reserve, X corresponding to the formula (I) below;

$$X = \frac{10 \times V_A}{RA \times C} \tag{I}$$

in which $V_A$ corresponds to the volume of acid needed to neutralise the reserve alcalinity RA, and C is the anti-freeze concentration of the cooling liquid, expressed as a percentage.

**2.** The method for implementing a device according to claim 1 consisting in measuring, consecutively,

a) the protection temperature of said liquid,
b) the pH of said liquid
c) and the reserve alcalinity RA of said liquid using a visual acid-basic colorimetric test,

said method being **characterised in that** said colorimetric test consists in sampling, in a graduated test tube, a volume X of said cooling liquid corresponding to the protection temperature measured, adding from 5-15 ml water to said test tube, and then introducing from 2 to 8 ml of a 0.05 to 0.2 N strong acid, adding several drops of colouring agent and then looking for a change in colour.

**3.** The method according to claim 2, **characterised in that** said strong acid is chosen from the group consisting of hydrochloric acid, sulphuric acid, nitric acid, hydrochloric acid being preferred.

**4.** The method according to claim 2, **characterised in that** said colouring agent has a range of pH turning points between 3 and 4.6.

**5.** The method according to one of claims 2-4, **characterised in that** the colouring agent is chosen from bromophenol blue, bromochlorophenol blue, Congo red and methyl orange.

**6.** The method according to claim 2, **characterised in that** each step in said sequence of operations (a) to (c) allows the cooling liquid to be accepted or refused, a cooling liquid with acceptable characteristics being the one which has successively passed steps a, b and c in this order.

ECHANTILLON

1

Tp

Tp > Tpo

2

Tp < Tpo

3

pH

pH < 6.6

4

pH > 6.6

5

RA

jaune

6

bleu

7

OK

FIG. 1